Europäisches Patentamt

(19) European Patent Office

Office européen des brevets

(11) Numéro de publication: **0 011 560 B1**

## (12) FASCICULE DE BREVET EUROPEEN

(45) Date de publication du fascicule du brevet:
19.01.83

(51) Int. Cl.³: **C 12 N 15/00**

(21) Numéro de dépôt: **79400849.0**

(22) Date de dépôt: **13.11.79**

(54) Vecteurs appropriés à l'insertion dans leurs génomes de fragments d'ADN étrangers, selon l'une quelconque des phases de traduction possibles, et moyens pour leur fabrication.

(30) Priorité: **13.11.78 FR 7832041**

(43) Date de publication de la demande:
**28.05.80 Bulletin 80/11**

(45) Mention de la délivrance du brevet:
**19.01.83 Bulletin 83/3**

(84) Etats contractants désignés:
**AT BE CH DE GB IT NL**

(73) Titulaire: **INSTITUT PASTEUR, 25-28, rue du Docteur Roux, F-75724 Paris Cedex 15 (FR)**
Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Tiollais, Pierre, 16, rue de la Glacière, F-75013 Paris (FR)**

(74) Mandataire: **Gutmann, Ernest et al, Cabinet Plasseraud 84, rue d'Amsterdam, F-75009 Paris (FR)**

(56) Documents cités:
**LA RECHERCHE, no. 94, novembre 1978, vol. 9, pages 1021—1023. O. MERCEREAU-PUIJALON, »Des bactéries productrices de protéines animales«.\* Page 1022, rème colonne, dernier paragraphe\***
**NUCLEIC ACIDS RESEARCH, vol. 5, no. 12, décembre 1978. P. CHARNAY et al. »Baxteriophage lambda and plasmid vectors, allowing fusion of cloned genes in each of the three translational phases«, pages 4479—4494**

Le dossier contient des informations techniques présentées postérieurement au dépôt de la demande et ne figurant pas dans le présent fascicule.

## Vecteurs appropries a l'insertion dans leurs génomes de fragments d'ADN étrangers, selon l'une quelconque des phases de traduction possibles, et moyens pour leur fabrication

L'invention est relative à des vecteurs ou groupes de vecteurs modifiés, notamment du type phage ou plasmide, dans le génome desquels peut être inséré, par fusion génétique, de préférence in vitro, un fragment d'ADN étranger susceptible de coder la production d'une protéine déterminée, procaryote ou eucaryote, ces phages étant construits de telle façon qu'ils permettent l'expression de ce fragment (gène ou tout autre ADN, par exemple celui résultant de la transcription enzymatique d'une ARN messager).

Elle concerne plus particulièrement des vecteurs de ce type qui contiennent un fragment d'opéron bactérien comportant au moins le promoteur et au moins une partie de gène ou de l'un des gènes qui lui est associé dans cet opéron bactérien, ce gène ou cette partie de gène comprenant un site de reconnaissance spécifique par une enzyme de restriction, telle que EcoRI, de préférence à l'exclusion de tout autre site semblable dans ce même vecteur. De préférence, le fragment du genre en question est originaire de l'opéron lactose et le gène ou fragment de gène provient du gène Z. Après ouverture in vitro de ce vecteur à l'aide de cette enzyme de restriction, on peut y insérer le fragment d'ADN étranger susdit, sous réserve qu'il ait, si besoin, été pourvu au préalable des extrémités cohésives correspondantes qui permettent alors leur appariement, notamment sous l'effet d'une ADN-ligase, avec les parties antérieurement séparées du vecteur en question.

Il a alors été constaté que le promoteur de l'opéron bactérien, présent ou inséré dans le vecteur était apte à fournir un signal suffisant à permettre le déclenchement de l'expression dans une bactérie dans laquelle ce vecteur aura préalablement été introduit, des gènes qui lui sont associés, donc y compris celui du fragment étranger, expression qui se manifeste alors par la production par la bactérie d'une protéine hybride contenant une séquence polypeptidique correspondant à la partie du gène de l'opéron bactérien, plus particulièrement une séquence correspondant au moins aux premiers acides aminés de la $\beta$-galactosidase dans le cas de l'opéron lactose, associée à celle codée par le fragment étranger. Ainsi a-t-il été possible récemment de réaliser l'expression par une bactérie de divers gènes eucaryotes, notamment ceux de la somatostatine, de la proinsuline et de l'ovalbumine de poulet.

Il faut cependant souligner que cette opération ne peut être menée favorablement qu'à condition que soit réalisé l'enchainement correct — en phase — dans le vecteur du gène de l'opéron bactérien et de l'ADN étranger. On sait en effet que le codage des acides aminés successifs d'une protéine fait intervenir des triplets successifs de paires de bases successives (codons) de l'ADN, de sorte qu'il faut s'assurer que la traduction du fragment d'ADN étranger, qui ne fait qu'enchaîner celle préalablement déclenchée du fragment de gène de l'opéron bactérien, à partir de leur point de jonction commun, se fasse selon le cadre de lecture normal du gène étranger, donc à partir des codons correspondant effectivement à la protéine recherchée, et non de ceux qui sont décalés soit d'une, soit de deux paires de bases vis-à-vis du point d'initiation normal de la traduction de la protéine étrangère. Cette opération a été réalisée avec succès dans le cas des exemples précédemment mentionnés, grâce à la connaissance que l'on avait des emplacements relatifs tant du fragment du gène Z (qui comprenait notamment les huit premiers triplets correspondant aux huit premiers amino-acides de la $\beta$-galactosidase) que du gène codant la protéine recherchée dans certains des vecteurs qui ont été utilisés, plus particulièrement pour la pro-insuline et l'ovalbumine.

La réalisation d'un tel enchaînement avec d'autres ADN étrangers peut être extrêmement délicate, voire impossible avec les mêmes vecteurs. Dans ce cas, à ce jour évidemment encore le plus fréquent, cette difficulté ne pourra même pas être appréciée de façon exacte, faute de connaître avec précision soit la position dans le fragment d'ADN du gène codant la protéine recherchée, soit même la nature de la protéine que ce fragment est apte à coder dans sa cellule d'origine.

L'invention a pour but de précisément fournir des moyens qui permettent de remédier à de telles difficultés, notamment des vecteurs ou groupes de vecteuers dans lesquels l'utilisateur puisse puiser pour rèaliser l'enchaînement approprié, ou qu'il puisse mettre en oeuvre simultanément pour déterminer rapidement la nature de celui qui assurera la traduction conforme de l'ADN étranger préalablement incorporé dans son génome.

Le vecteur selon l'invention qui est dérivé d'un vecteur préexistant, tel qu'il a été défini plus haut, est caractérisé en ce que l'emplacement du site de reconnaissance de l'enzyme de restriction considéré est décalé vis-à-vis de celui de ce même site dans le vecteur préexistant, ou encore vis-à-vis du point d'initiation de la traduction du gène ou fragment de gène associé à son promoteur, par l'intermédiaire d'un groupe formé, soit de deux paires de bases, soit d'une ou quatre paires de bases, le cas échéant associé dans chaque cas à un multiple entier de triplets supplémentaires, les paires de bases de ce groupe pouvant être quelconques, cependant à l'exclusion de celles qui, dans l'ordre dans lequel elles se trouveraient placées, formeraient au moins un codon »non-sens«, c'est-à-dire l'un des trois codons sur les soixante-quatre possibles, dont la fonction dans le code génétique est précisément d'interrompre la traduction.

Des vecteurs préférés selon l'invention sont ceux dans lesquels le promoteur est celui de l'opéron lactose et le gène ou partie de gène qui lui est associé est constitué par le gène Z ou un fragment de ce gène Z, tel que celui qui est apte à coder les huit premiers amino-acides de la β-galactosidase, le site de restriction considéré étant alors de préférence un site EcoRI.

Les décalages peuvent également s'exprimer vis-à-vis du point fixe dans le vecteur considéré, avantageusement celui correspondant au point d'initiation de la traduction. Partant par conséquent d'un vecteur dans lequel la première paire de bases du site de l'enzyme de restriction est séparé de ce point d'initiation par un nombre entier de triplets correspondant aux premiers. amino-acides de la protéine ou partie de protéine normalement codée par le gène ou partie de gène considéré (tel que le gène Z ou les huit premiers triplets de celui-ci) on obtient les deux autres vecteuers tels qu'ils ont été définis ci-dessus, par décalage du site de reconnaissance par l'enzyme de restriction vis-à-vis du point d'initiation de la traduction, par insertion entre ce dernier et cette première paire de bases de groupes formés respectivement de deux paires de bases et d'une ou quatre paires de bases (auxquelles peuvent, le cas échéant, en outre être associés des multiples entiers de triplets supplémentaires), les groupes insérés devant naturellement répondre à la condition exprimée plus haut en ce qui concerne le choix des paires de bases insérées. De préférence, ces insertions sont réalisées à proximité immédiate de la première paire de bases dudit site de reconnaissance.

L'invention concerne également les ensembles ou jeux de vecteuers qui peuvent ainsi être consitutés et notamment présentés sous forme de trousses ou »kits« de trois vecteuers, se distinguant essentiellement entre eux en ce que, l'un de ces vecteurs étant caractérisé par le fait que la première paire de bases de son site de reconnaissance par l'enzyme de restriction se trouve à une distance du point d'initiation de la traduction correspondant à un multiple entier de triplets, les premières paires de bases des sites de reconnaissance des deux autres vecteurs sont respectivement décalées vis-à-vis du même point d'initiation d'une distance correspondant à des nombres entiers de triplets auxquels viennent s'ajouter respectivement, d'une part, deux paires de bases et, d'autre part, une ou quatre paires de bases.

Avantageusement, ces trois vecteurs comportent respectivement des mutations distinctes permettant leur reconnaissance rapide dans des opérations de recombinaison génétique les mettant en oeuvre tous les trois.

L'invention met ainsi à la disposition du technicien les moyens lui permettant de réaliser efficacement des recombinaisons génétiques notamment in vitro, susceptibles de fournir le vecteur de recombinaison dans lequel le fragment étranger inséré pourra ensuite être

exprimé par mise en oeuvre du cadre de lecture correcte par les bactéries préalablement transformées avec de tels vecteurs.

Plus particulièrement, l'invention est applicable dans chacune des alternatives suivantes. L'invention permet le choix du vecteur approprié dans le cas où l'on connait la séquence primaire du gène à insérer, de sorte qu'il suffit de choisir celui des trois vecteurs qui permettra, à l'occasion de la traduction, de respecter la phase de lecture du gène inséré. Au contraire, dans le cas où l'on ne connaît pas la structure première du gène à insérer, on produira l'insertion de ce gène dans les trois vecteurs en question. C'est alors celui de ces trois vecteurs qui permettra l'expression complète du gène inséré qui constituera le vecteur approprié à la phase de lecture correcte de ce gène. En effet, il est en toute probabilité vraisemblable que les expressions erronées obtenues par l'intermédiaire de deux autres vecteurs se traduiront en général par la production de protéines de poids moléculaires beaucoup plus faibles, en tous cas ayant des propriétés biologiques différentes. En effet, on peut admettre que, le plus souvent, la traduction se trouvera interrompue du fait de la présence d'un codon »non-sens« (statistiquement en moyenne trois sur soixante-quatre codons) dans le cas de lecture erronée, en raison de l'enchaînement horsphase du gène étranger à la partie de gène de l'opéron bactérien du vecteur.

L'invention concerne également les fragments d'ADN ci-après dénommés »fragments d'insertion«, appropriés à la fabrication de vecteuers du type sus-défini comportant au moins le promoteur et une partie du gène ou de l'un des gènes qui lui est associé dans l'opéron bactérien correspondant, ces fragments étant caractérisés en ce que deux de ces fragments se distinguent du troisième par un décalage de leurs extrémités cohésives vis-à-vis du point d'initiation de la lecture de gène ou partie de gène correspondant mettant en jeu des distances correspondant respectivement à des groupes, d'une part, de deux paires de bases et, d'autre part, d'une ou quatre paires de bases, auxquels s'ajoutent, le cas échéant, des distances correspondant à des multiples entiers de triplets supplémentaires, les paires de bases de ces groupes pouvant être quelconques, cependant à l'exclusion de celles qui, dans l'ordre dans lequel elles se trouveraient placées, formeraient au moins un codon »non-sens«.

Comme dans le cas des vecteurs, le promoteur et le gène correspondant associé sont de préférence respectivement le promoteur et le gène Z de l'opéron lactose, l'extrémité cohésive considérée étant une extrémité cohésive EcoRI.

L'invention concerne encore un procédé pour fabriquer à partir d'un fragment d'insertion initial comportant au moins une extrémité cohésive correspondant à une enzyme de restriction déterminée, un autre fragment dont la constitution entraîne, après insertion dans un même vecteur, dans des conditions semblables à celles de l'insertion du fragment initial, un décalage d'une phase au niveau de son expression dans une bactérie, ce procédé consistant à rogner le fragment saillant que forme l'un des brins de l'ADN au niveau de ladite extrémité cohésive, notamment au moyen d'une endonucléase appropriée, à recombiner aus moyen d'une ligase le fragment ainsi modifié, au niveau de l'extrémité franche formée avec un autre fragment d'ADN ayant un site de reconnaissance pour cette même enzyme de restriction et comportant également une extrémité franche, celle-ci étant séparée de la première paire de bases intervenant dans le site de reconnaissance par un nombre de paires de bases correspondant à la somme de deux ou quatre paires de bases et, le cas échéant, d'un multiple entier de triplets supplémentaires et enfin à traiter le fragment d'insertion ainsi obtenu avec cette même enzyme de restriction, afin de reformer l'extrémité cohésive en question.

Il va de soi que l'on peut encore former un fragment d'insertion dont l'utilisation conduira à la troisième possibilité de lecture possible du vecteur modifié par la bactérie, en répétant une nouvelle fois toutes ces opérations.

Un tel »autre fragment« tel que ci-dessus défini consiste par exemple en un court fragment dénommé ci-après »linker« de formule

$$P5' \text{ GGAATTCC}$$
$$\text{CCTTAAGG} 5'P$$

dans laquelle A représente l'adénine, T la thymine, C la cytosine et G la guanine.

Les insertions des trois fragments à phases décalées ainsi obtenues dans un phage ou dans un plasmide, puis les insertions supplémentaires d'un fragment d'ADN étranger dans les trois types de vecteurs susceptibles d'être ainsi obtenus, conduisent à des vecteurs susceptibles de conduire à des traductions déphasées l'une par rapport à l'autre des fragments étrangers qu'ils comportent respectivement. Les cadres de lecture correspondant à ces trois phases peuvent notamment être illustrés comme suit, au niveau des nucléotides intervenant dans les sites de reconnaissance EcoRI de tels vecteuers

$$\Phi 1 \ 5' \ G \downarrow A \ A \ T \ T \ C \ 3',$$

$$\Phi 2 \ 5' \ G \downarrow A \ A \ T \ T \ C \ 3',$$

$$\Phi 3 \ 5 \ G \downarrow A \ A \ T \ T \ C \ 3'.$$

Il va de soi qu'il ne s'agit que d'un exemple de fragment qui peut être utilisé pour des opérations de ce type.

Les fragments d'insertion obtenus peuvent alors être insérés dans des vecteurs, phages ou plasmides, par toute technique en soi déjà connue.

D'autres caractéristiques de l'invention apparaîtront encore au cours de la description qui suit d'exemples de fabrication de fragments d'insertion et vecteurs préférés mettant en oeuvre les caractéristiques de l'invention. Il sera fait référence aux dessins dans lesquels:

- la fig. 1 est un schéma illustrant le procédé selon l'invention,
- la fig. 2 est un schéma illustrant les étapes principales de la recombinaison d'un tel fragment d'insertion avec un vecteur,
- la fig. 3 est un schéma de différents vecteurs que l'invention permet de constituer ?t enfin
- la fig. 4 est une représentation schématique de la fabrication d'un fragment d'insertion conforme à un mode de mise en oeuvre préféré de l'invention.

Les nombres entre parenthèses dans la description qui suit renvoient à la bibliographie en fin de description.

## Materiels et methodes

### Souches bactériennes et bactériophages

Toutes les souches bactériennes utilisées sont issues de la souche E. coli K12: Il s'agit de la souche MM294 qui porte le plasmide pOP203 (UV5) C.N.Ć.M. n° I-066; la souche C600 recBC $rk^-mk^-$ C.N.C.M. n° I-067; les souches YMC (SupF) C.N.C.M. n° I-068 et 3000X74 (lac sup°) C.N.C.M. n° I-069; phage $\lambda$ZQS-EcoRI (C.N.C.M. n° I-055).

### Produits chimiques

Les produits chimiques suivants ont été utilisés: Xgal = 5-bromo-4-chloro-3-indolyl-$\beta$-D galactoside (Bachem). Mélibiose (Baker). Agarose (Sigma). Acrylamide (Serva). 32P $\gamma$ ATP (New England Nuclear). Octadéoxyribonucléotide 5'-OH-GGAATTCC-OH-3' (Collaboration Research). Diméthyl-sulfate (Aldrich Chemical). Hydrazine (Eastman Organic Chemicals). Pipéridine (BDH Biochemicals).

### Réactions enzymatiques

Les hydrolyses par l'endonucléase HindIII (Biolabs) ont été faites en tampon Tris HCl: 6,6 mM pH 7,5, MgCl2: 6,6 mM, $\beta$-mercaptoéthanol: 6,6 mM, NaCl: 6 mM. Les hydrolyses par les endonucléases HpaII et .AluI ont été faites en tampon Tris HCl: 6,6 mM, pH 7,5, MgCl2: 6,6 mM, $\beta$-mercaptoéthanol: 6,6 mM, NaCl: 6,6 mM. Les hydrolyses par l'endonucléase EcoRI et la ligature des extrémités cohésives par la polynucléotide ligase de T4 ont été décrites précédemment (12). La phosphatase alkaline (PL Laboratories), la DNase I purifiée par électrophorèse (Worthington) et la phosphodiestérase de venin de serpent (Worthington) ont été utilisées dans le tampon Tris HCl: 10 mM, pH 8,5, MgCl2: 10 mM (13). La réaction de phosphorylation en 5' avec du 32P γ ATP par la polynucléotide kinase (PL Laboratories) a été faite comme décrit par Maxam et Gilbert (14). L'hydrolyse par l'endonucléase Sl a été faite dans le tampon NaCOOCH3: 25 mM, pH 4,5, ZnSO4: 1 mM, NaCl: 125 mM pendant 1 heure à 25°C. La quantité adéquate d'enzyme a été déterminée par l'analyse de l'extrémité nucléotidique 5' terminale du fragment EcoRI 203 traité par des quantités croissantes d'enzyme.

### Ligature d'extrémités franches d'octadéoxyribonucléotides (»EcoRI linkers«) aux fragments d'ADN

1 µg d'»EcoRI linker« a été marqué à l'extrémité 5' phosphate par 25 µCi de 32P γ ATP (activité spécifique 3 mCi/mM) à l'aide de 2 unités de polynucléotide kinase dans 150 µl de tampon Tris HCl: 50 mM, pH 9,5, MgCl2: 10 mM, DTT: 5 mM pendant une heure à 37°C. Après addition de 0,03 mM d'ATP, 2 unités de polynucléotide kinase ont été ajoutées et l'incubation a été continuée encore une heure à 37°C. Le mélange a été lyophilisé et le résidu solide dissous dans 20 µl de tampon Tris HCl: 6,6 mM, pH 7,6, MgCl2: 6,6 mM, ATP: 1 mM, DTT: 10 mM qui contenait environ 10 ng de fragments d'ADN EcoRI 203 (UV5) préalablement traités par l'endonucléase S1. Après addition de 10 unités de polynucléotide ligase de T4, le mélange est incubé pendant 48 heures à 14°C.

### Autres techniques

Les électrophorèses sur gel analytiques et préparatives ont été faites selon Adams (15). L'élution des fragments de DNA a été réalisée comme décrit par Maxam et Gilbert (14). L'électrophorèse sur papier 3 MM à pH 3,5 a été réalisée comme décrit par Barell (13). La séquence du DNA a été réalisée comme décrit par Maxam et Gilbert (14). La filtration sur le tamis moléculaire connu sous la désignation SEPHADEX G100 a été réalisée selon Sanger (16) dans des pipettes sérologiques CORNING de 1 ml. La multiplication des bactériophages et les corisements génétiques ont été réalisés dans la souche bactérienne YMC, la purification de bactériophages, la transfection dans E. coli C600 rk⁻mk⁻ recBC. Les électrophorèses ont été réalisées sur gels d'agarose. Xgal, tétracycline et ampicilline ont été utilisés en milieu solide L respectivement aux concentrations suivantes: 40 mg/litre, 15 mg/litre et 100 mg/litre.

### Confinement

Les expériences ont été faites dans des conditions L1 (PI) et BI (EK1).

### Resultats

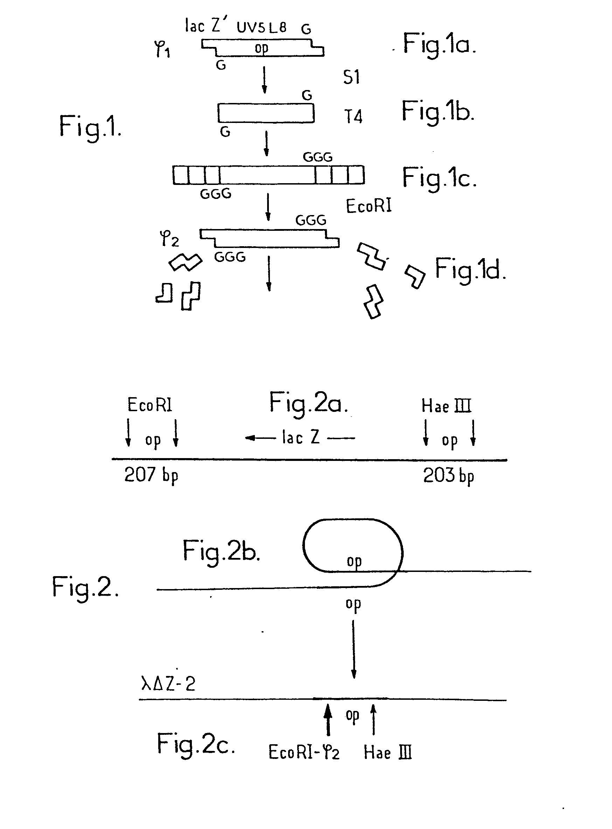

#### Modifications chimiques des extrémités du fragment de DNA EcoRI 203 (UV5) (fig. 1)

Le procédé d'addition de deux paires de bases à chaque extrémité du fragment de DNA EcoRI 203 (UV5) a été le suivant:

1) Traitement d'un fragment lac Z' UV5L8G (fig. 1a) par l'endonucléase Sl de telle façon à produire des extrémités franches possédant

$$G$$
$$C\ 5'P$$

(fig. 1b);
2) Addition par la T4 DNA ligase (T4 dans la fig. 1) du »linker« EcoRI

$$P5'\ GGAATTCC$$
$$CCTTAAGG5'P$$

à l'extrémité du fragment de DNA;
3) Digestion du nouveau fragment de DNA (schématisé dans la fig. 1c) par l'endonucléase EcoRI, ce qui engendre un fragment de DNA possédant deux paires de base supplémentaires

$$GG$$
$$CC$$

à chaque extrémité EcoRI (fig. 1d). Ce dernier fragment de DNA est appelé EcoRI 207 (UV5) fragment. Le fragment de DNA EcoRI 211 (UV5) a été construit par le même procédé.

125 µg de plasmide pOP203 qui porte le fragment EcoRI 203 (UV5) ont été hydrolysés par l'endonucléase EcoRI. Le DNA a alors été traité par l'endonucléase Sl. Après marquage à l'extrémité 5' par l'ATP γ 32P en utilisant la polynucléotide kinase, le fragment EcoRI 203 (UV5) a été préparé par électrophorèse dans un gel de polyacrylamide à 8%. Pour tester l'efficacité de l'endonucléase Sl, un aliquot de DNA a été utilisé pour déterminer la nature du nucléotide 5' terminal. Il était comme attendu essentiellement constitué de cytosine.

Les linkers EcoRI ont été ligaturés au fragment de DNA (voir Matériels et Méthodes). L'analyse électrophorétique d'aliquots prélevés avant et après le traitement EcoRI, a montré que les linkers étaient attachés au fragment de DNA. Le fragment de DNA digéré par EcoRI a été séparé des linkers par chromatographie sur colonne de SEPHADEX G100. Le fragment a été élué dans un volume total de 100 µl.

Une partie du fragment de DNA modifié, dans 15 µl, a été alors ligaturé in vitro au plasmide pBR322 (17) digéré par EcoRI. Ce DNA a été utilisé pour transformer la souche bactérienne MM294. Environ 8% des colonies étaient bleues sur milieu L Xgal, tétracycline, ce qui indiquait qu'elles portaient le plasmide pBR322 avec l'opérateur lac.

### Analyse de l'extrémité lac Z du fragment de DNA EcoRI 207 (UV5)

A partir de colonies bleues décrites dans la section précédente, 4 clones ont été amplifiés et la structure de leurs plasmides (pPC21, pPC22, pPC23 et pPC24) a été analysée. Le traitement de chaque plasmide par l'endonucléase EcoRI a produit un fragment de DNA dont la mobilité electrophorétique (dans un gel d'acrylamide à 8%) est légèrement moindre que celle du fragment EcoRI 203 (UV5) initial. Cette bande correspond au fragment EcoRI 207 (UV5).

Pour être certain que les extrémités EcoRI du fragment EcoRI 207 ont été déplacées de deux paires de bases, la séquence nucléotidique de l'extrémité lac Z du fragment EcoRI 207 (UV5) a été déterminée. 100 µg des plasmides pOP203, pPC21 et pPC22 ont été digérés par l'endonucléase EcoRI. Pour suivre la purification du fragment de DNA, 5 µg de chaque plasmide ont été marqués en 5' par l'ATP $\gamma$ 32P et additionnés au reste de l'échantillon. Les DNA ont été précipités par l'éthanol et le culot dissous dans 100 µl d'acétate de sodium 30 mM. Les fragments EcoRI 203 (UV5) et EcoRI 207 (UV5) ont été purifiés par électrophorèse dans un gel de polyacrylamide à 8%. Après élution du gel, les DNA ont été marqués par de l'ATP $\gamma$ 32P à haute activité spécifique. Après digestion par l'endonucléase HpaII, le fragment de DNA radioactif le plus court, qui correspond à l'extrémité du gène lac Z a été purifié par électrophorèse dans un gel de polyacrylamide à 8%. La séquence nucléotidique de ces fragments de DNA a été effectuée selon la méthode de Maxam et Gilbert. Comme prévu, la séquence des plasmides pPC21 et pPC22 a montré que deux paires de bases GC ont été insérées immédiatement avant le site de restriction EcoRI et donc que l'extrémité lac Z des deux fragments EcoRI 207 (UV5) possède la phase de traduction qui est la phase $\Phi$2 définie antérieurement (fig. 2).

### Construction du fragment de DNA EcoRI 211 (UV5) et vérification de sa structure

Le fragment de DNA EcoRI 211 (UV5) a deux paires de bases supplémentaires à chaque extrémité par rapport au fragment EcoRI 207 (UV5). Ce fragment de DNA a été obtenu à partir du fragment EcoRI 207 (UV5) par une série d'étapes identiques à celles qui ont fourni le fragment de DNA EcoRI 207 (UV5) à partir du fragment EcoRI 203 (UV5). La séquence nucléotidique de l'extrémité lac Z du fragment EcoRI 211 (UV5) a été déterminée à partir de trois plasmides (pPC31, pPC32 et pPC33). L'extrémité EcoRI a quatre paires de bases GC supplémentaires par rapport au fragment initial EcoRI 203 (UV5) et correspond à la phase $\Phi$3 (fig. 2).

### Construction des bactériophages λ𝛥Z1, λ𝛥Z2 (phase $\Phi$2) et λ𝛥Z3 (phase $\Phi$3)

Le bactériophage λ𝛥Z1 est fabriqué de la façon suivante à partir du bactériophage λZQS-EcoRI déposé sous le n° I-055 à la C.N.C.M.

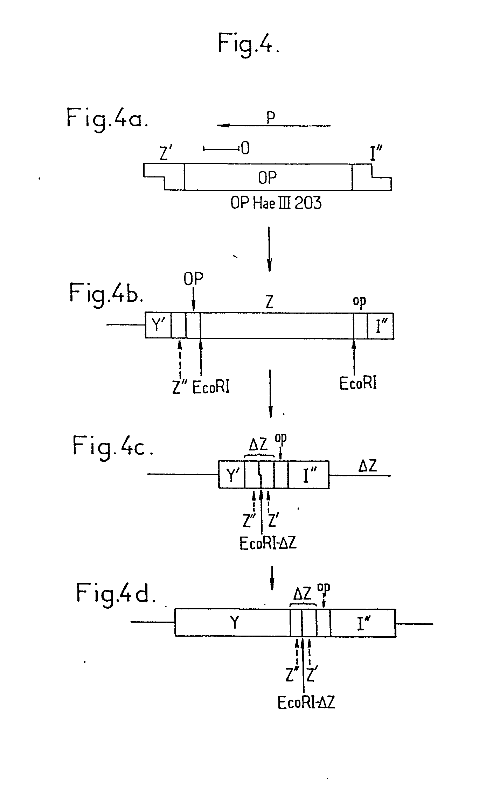

Le phage λ𝛥Z1 se distingue du précédent essentiellement par une délétion dans les conditions décrites ci-après à l'aide des schémas illustratifs des fig. 4a à 4d.

Partant du phage λ𝛥ZQS-EcoRI, le fragment OP Hae III 203 (schéma 4a), portant deux extrémités EcoRI, a été inséré dans le site EcoRI-Z (par la technique décrite par Backman, K., et Coll., Proc. Nat. Sci. [1976], 73, pp. 4174—4178). Il comporte aussi, de part et d'autre d'une partie centrale OP, un fragment Z' provenant du début du gène Z et correspondant aux sept premiers amino-acides susceptibles d'être codés par le gène Z, et un fragment terminal I" du répresseur de l'opéron lactose. Lorsque le fragment OP Hae III 203 (représenté à échelle réduite dans le schéma 4b) est inséré dans le même sens que le fragment OP homologue à proximité du fragment terminal Z" du gène Z du phage (visé par la référence »op« dans le schéma 4b et à proximité d'un fragment terminal I" du gène I du répresseur de l'opéron lactose), une recombinaison intramoléculaire crée une délétion (fig. 4c) de la presque totalité du gène Z. Ceci permet, par ailleurs, de créer un site EcoRI (site EcoRI-𝛥Z) à la place du site Hae III, très près de l'origine du gène Z à un site correspondant au septième acide aminé de la β-galactosidase. Il ne subsiste finalement du gène Z initial que les fragments Z" et Z', de part et d'autre du site EcoRI-𝛥Z.

Le bactériophage λ𝛥Z2 a été construit comme le bactériophage λ𝛥Z1 (11), comme il est indiqué sur la fig. 2.

1) Insertion in vitro du fragment EcoRI 207 (UV5) dans le site EcoRI lac Z du génome de λplac5-1 UV5 (fig. 2a) et
2) recombinaison génétique intramoléculaire (schématisée par la fig. 2b).

La première étape a donné des bactériophages lac⁻ avec le phénotype $\alpha^+ \beta^+ \omega^-$ et la seconde étape a donné des bactériophages lac⁻ ($\alpha^- \beta^- \omega^-$) appelés λ𝛥Z2. Le tracé électrophorétique des fragments de DNA du bactériophage λ𝛥Z2 (fig. 2c) après digestion mixte avec EcoRI + HindIII, était identique au tracé obtenu avec le DNA λ𝛥Z1. Ceci a confirmé la recombinaison génétique intramoléculaire.

Les bactériophages λ𝛥Z3, λY3 et λY3ZQS ont été construits de la même manière. λ𝛥Z3 a été obtenu par insertion in vitro du fragment EcoRI 211 (UV5) dans le site de restriction du $\lambda$plac5-1, suivie d'une recombinaison génétique intramoléculaire. Les bactériophages sont représentés sur la fig. 4.

### Construction des plasmides pPC$\Phi$1, pPC$\Phi$2 et pPC$\Phi$3

Ces plasmides ont été construits pour étendre le système des trois phases à des vecteurs plasmidiques. Les trois plasmides ont été obtenus par substitution du petit fragment EcoRI-HindIII du plasmide pBR322 (C.N.C.M. n° I-065) par le fragment EcoRI-HindIII des génomes $\lambda\Delta$Z1, $\lambda\Delta$Z2 et $\lambda\Delta$Z3.

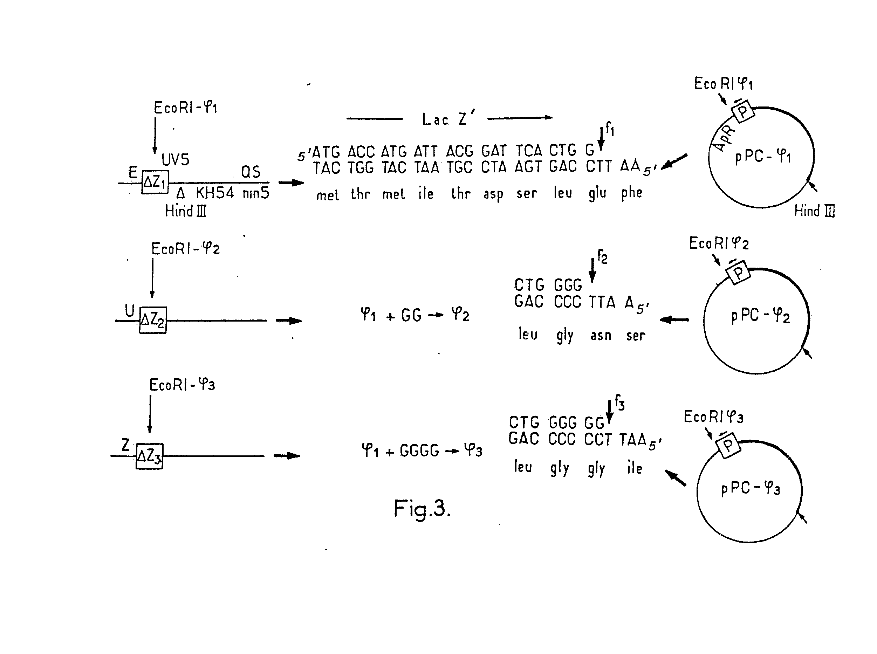

Le procédé expérimental a été le suivant: 500 µg de chaque DNA $\lambda\Delta$Z1, $\lambda\Delta$Z2 et $\lambda\Delta$Z3 ont été hydrolysés par les enzymes EcoRI et HindIII, après mélange avec des aliquots de 100 µg de DNA pBR322 digérés de la même façon. Après traitement par la ligase, les trois échantillons de DNA ont été utilisés pour transformer la souche MM294. Des colonies bleues sur milieu L Xgal ampicilline ont été isolées. Pour chaque type de vecteur, les plasmides de trois clones ont été analysés. Après digestion par les endonucléases EcoRI et HindIII, l'analyse électrophorétique a donné dans tous les cas deux bandes de DNA, l'une étant le fragment EcoRI-HindIII provenant des bactériophages, l'autre étant le fragment plasmidique EcoRI-HindIII. Les résultats sont montrés pour un plasmide de chaque type, appelés respectivement pPC$\Phi$1, pPC$\Phi$2 et pPC$\Phi$3. Pour vérifier que ces trois plasmides correspondent aux trois phases, l'analyse suivante a été faite. Le DNA de chacun des trois plasmides a été digéré par l'endonucléase EcoRI, l'extrémité a été déphosphorylée et marquée par l'ATP $\gamma$ 32P, et finalement digérée par l'endonucléase AluI, qui coupe le promoteur lac très près de l'extrémité lac Z. L'analyse électrophorétique du fragment dans un gel de polyacrylamide à 20% a montré qu'il a la taille attendue. Les trois plasmides pPC$\Phi$1, pPC$\Phi$2 et pPC$\Phi$3 ont un seul site EcoRI situé au début du gène lac Z et permettent l'insertion d'un gène dans ce site dans une des trois phases par rapport au site d'initiation de traduction du gène lac Z (fig. 3).

Les structures des vecteurs, phages ou plasmides obtenus sont brièvement rappelés à la fig. 3. L'indication Lac Z' correspond à la séquence du gène s'étendant entre le site d'initiation de la traduction et le site EcoRI. L'addition de deux paires de base GC à cette séquence transforme le site EcoRI-$\Phi$1 en un site EcoRI-$\Phi$2. L'addition encore de deux paires de base supplémentaires GC conduit au site EcoRI-$\Phi$3. Les bactériophages obtenus figurent à la partie gauche du dessin, les plasmides obtenus à la droite du dessin.

Les flèches f1, f2 et f3 sont illustratives des points de départ de la traduction du gène étanger susceptible d'être inséré dans ces vecteurs.

Comme il va de soi et comme il résulte d'ailleurs déjà de ce qui précède, l'invention ne se limite nullement à ceux de ses modes d'application et de réalisation qui ont été plus spécialement envisagés. En particulier, les groupes d'insertion qui sont à l'origine des décalages en question ne sont pas nécessairement prévus à proximité immédiate des sites de reconnaissance sus-visés; ils pourraient être introduits ailleurs, plus particulièrement dans le fragment de gène Z, pour autant que cette insertion ne perturberait pas le déclenchement de la traduction de ces gènes sous l'effet du promoteur; comme il a déjà été indiqué, d'autres opéron bactériens ou parties d'opérons bactériens comme l'opéron lactose ou parties d'opéron lactose peuvent être mis en oeuvre dans le cadre de l'invention; à titre d'exemple, on peut citer les opérons tryptophane, maltose, histidine, etc.

### Bibliographie

1. Struhl, K., Cameron, J. R. and Davis, R. W. (1976), Proc. Natl. Acad. Sci. USA 73, 1471–1475.
2. Ratzkin, B. and Carbon, J. (1977), Proc. Natl. Acad. Sci., USA 74, 487–491.
3. Vapnek, D., Hautala, J. A., Jacobson, J. W., Giles, N. H. and Kushner, S. R. (1977), Proc. Natl. Acad. Sci., USA 74, 3508–3512.
4. Itakura, K., Hirose, T., Crea, R., Riggs, A. D., Heyneker, H. L., Bolivar, F. and Boyer, H. W. (1977), Science 198, 1056–1063.
5. Villa-Komaroff, L., Efstratiadis, A., Broome, S., Lomedico, P., Tizard, R., Naber, S. P., Chick, W. L. and Gilbert, W. (1978), Proc. Natl. Acad. Sci., USA 75, N° 8, 3727–3731.
6. Mercereau-Puijalon, O., Royal, A., Cami, B., Garapin, A., Krust, A., Gannon, F. and Kourilsky, Ph. (1978), Nature 275, 505–510.
7. Chow, L. T., Gelinas, R. E., Broker, T. R. and Roberts, R. J. (1977), Cell 12, 1–8.
8. Aloni, Y., Dhar, R., Laub, O., Horowitz, M. and Khoury, G. (1977), Proc. Natl. Acad. Sci., USA 74, 3686–3690.
9. Marx, J. L. (1977), Science 197, 853–923.
10. Jeffreys, A. J. and Flavell, R. A. (1977), Cell 12, 1097–1108.
12. Tiollais, P., Perricaudet, M., Pettersson, U. and Philipson, L. (1976), Gene 1, 49–63.
13. Barell, B. G. (1971), Proc. in Nucl. Acid. Res. 2, 751–779.
14. Maxam, A. M. and Gilbert, W. (1977), Proc. Natl. Acad. Sci., USA 74, 560–564.
15. Adams, J. M., Jeppesen, P. G. N., Sanger, F. and Barrel, B. G. (1969), Nature 223, 1009–1014.
16. Sanger, F. and Coulson, A. R. (1975), J. Molec. Biol. 94, 441–448.
17. Bolivar, F., Rodriguez, R. L., Greene, P. J., Betlach, M. C., Heyneker, H. L., Boyer, H. W., Crossa, J. H. and Falkow, S. (1977), Gene 2, 95 – 113.
18. Les Recombinaisons génétiques in vitro. Le progrès scientifique 191, nov-déc. 1977.
20. Moir, A. and Brammar, W. J. (1976), Molec. Gen. Genet. 149, 87 – 89.
21. Donoghue, D. J. and Sharp, P. A. (1976), Gene 1, 209 – 227.

## Revendications

1. Ensemble de vecteurs comprenant trois vecteurs, dans lequel chacun de ces vecteurs contient un fragment comportant au moins le promoteur et au moins une partie du gène ou de l'un des gènes qui lui est associé dans l'opéron bactérien correspondant, le point d'initiation de la traduction et un site de reconnaissance par une enzyme de restriction déterminée, cet ensemble étant essentiellement caractérisé par le fait que les positions de ce site de reconnaissance sont respectivement décalées dans un second de ces vecteurs, par l'intermédiaire d'un groupe comprenant deux paires de bases et, le cas échéant, un nombre entier de triplets supplémentaires et, dans un troisième de ces vecteurs, par l'intermédiaire d'un groupe comprenant une ou quatre paires de bases et, le cas échéant, un nombre entier de triplets supplémentaires, vis-à-vis de la position de ce site dans le premier de ces vecteurs par rapport au susdit site d'initiation, les paires de bases contenues dans ces groupes pouvant être quelconques, cependant à l'exclusion de celles qui, dans l'ordre dans lequel elles seraient placées, formeraient au moins un codon »non-sens«.

2. Ensemble de vecteurs selon la revendication 1, caractérisé en ce que les trois vecteurs susdits comportent respectivement des mutations distinctes permettant leur reconnaissance rapide dans des opérations de recombinaison génétique les mettant en oeuvre tous les trois.

3. Ensemble de vecteuers selon la revendication 1 ou la revendication 2, caractérisé en ce que le susdit promoteur est celui de l'opéron lactose et que le gène susdit est le gène Z de ce même opéron.

4. Ensemble de vecteurs selon la revendication 3, caractérisé en ce que le susdit site de reconnaissance par une enzyme de restriction déterminée est un site EcoRI, ce site EcoRI étant unique dans chacun desdits trois vecteurs.

5. Ensemble de vecteurs selon la revendication 4, caractérisé en ce que dans l'un de ces vecteurs le site de l'enzyme de restriction est séparé du point d'initiation de la traduction par un nombre entier de triplets comportant les huit premiers triplets correspondant aux huit premiers aminoacides de la $\beta$-galactosidase, auxquels s'ajoute dans le second vecteur un groupe formé de deux paires de bases et dans le troisième vecteur une ou quatre paires de bases.

6. Ensemble de fragments d'ADN pour la fabrication d'un ensemble de vecteurs selon l'une quelconque des revendications 1 à 5, ces fragments comportant au moins le promoteur et une partie du gène ou de l'un des gènes qui lui est associé dans l'opéron bactérien correspondant, ces fragments étant caractérisés en ce que deux de ces fragments se distinguent du troisième par un décalage de leurs extrémités cohésives vis-à-vis du point d'initiation de la lecture du gène ou partie de gène correspondant, ces décalages étant produits dans les deux fragments par des groupes respectivement de deux paires de bases et d'une ou de quatre paires de bases, auxquels peuvent, le cas échéant, en outre être associés des nombres entiers de triplets supplémentaires, les paires de bases de ces groupes pouvant être quelconques, cependant à l'exclusion de celles qui, dans l'ordre dans lequel elles se trouveraient palcées, formeraient au moins un codon »non-sens«.

7. Ensemble de fragments d'ADN selon la revendication 6, caractérisé en ce que dans chacun d'entre eux le promoteur et le gène correspondant associé sont de préférence respectivement le promoteur et le gène Z de l'opéron lactose, les extrémités cohésives considérées étant des extrémités cohésives EcoRI.

8. Ensemble de fragments d'ADN selon la revendication 7, caractérisé en ce que la première base de l'extrémité cohésive est séparée du point d'initiation susdit par un nombre entier de triplets comprenant les huit premiers triplets, correspondant eux-mêmes aux huit premiers aminoacides de la $\beta$-galactosidase, auxquels s'ajoutent, dans le second fragment, un groupe formé de deux paires de bases et, dans le troisième fragment, d'une ou de quatre paires de bases.

9. Procédé pour fabriquer à partir d'un fragment d'insertion comportant au moins une extrémité cohésive correspondant à une enzyme de restriction déterminée, deux autres fragments dont les constitutions entraînent, après insertion dans un même vecteur, des décalages de phase distincts au niveau de son expression dans une bactérie, ce procédé étant caractérisé par les opérations qui consistent:

— à rogner le fragment saillant que forme l'un des brins de l'ADN au niveau de ladite extrémité cohésive, au moyen d'une endonucléase appropriée,

— à recombiner, au moyen d'une ligase, le fragment ainsi modifié, au niveau de l'extrémité franche formée avec un autre fragment d'ADN distinct déterminé ayant un site de reconnaissance pour cette même enzyme de restriction et comportant également une extrémité franche, celle-ci étant séparée de la première paire de bases intervenant dans le site de reconnaissance par un groupe de paires de bases contenant

— soit deux paires de bases et, le cas échéant, un nombre entier de triplets supplémentaires s'ajoutant aux précédentes deux paires de bases,

— soit quatre paires de bases et, le cas échéant, un nombre entier de triplets supplémentaires s'ajoutant aux précédentes quatre paires de bases;

les paires de bases contenues dans ce groupe pouvant être quelconques, cependant à l'exclusion de celles qui, dans l'ordre dans lequel elles seraient placées, formeraient au moins un codon »non-sens«,

— à traiter le fragment d'insertion modifié ainsi obtenu avec cette enzyme de restriction, afin de reformer l'extrémité cohésive en question et de former ainsi l'un des deux autres fragments comportant l'un des susdits décalages de phase distincts et

— enfin à répéter les opérations qui précèdent sur le fragment obtenu, pour finalement obtenir l'autre desdits fragments comportant l'autre desdits décalages de phase distincts.

**Patentansprüche**

1. Ein drei Vectoren umfassender Satz von Vectoren, in welchem jeder dieser Vectoren ein Fragment enthält, welches wenigstens den Promoter und wenigstens einen Teil des Gens oder des einen der Gene, mit welchem er im entsprechenden bakteriellen Operon assoziiert ist, den Ausgangspunkt der Translation und eine Erkennungsstelle für ein bestimmtes Restriktionsenzym umfaßt, wobei dieser Satz im wesentlichen dadurch gekennzeichnet ist, daß die Positionen dieser Erkennungsstelle in einem zweiten dieser Vectoren mittels einer zwei Basenpaare und gegebenenfalls eine ganze Zahl von zusätzlichen Tripletts umfassenden Gruppe, und, in einem dritten dieser Vectoren, mittels einer ein Basenpaar oder vier Basenpaare und gegebenenfalls eine ganze Zahl von zusätzlichen Tripletts umfassenden Gruppe gegenüber der Position dieser Erkennungsstelle in dem ersten dieser Vectoren, bezogen auf den obgenannten Ausgangspunkt der Translation, verschoben sind, wobei die in diesen Gruppen enthaltenen Basenpaare von beliebiger Art sein können, wobei jedoch solche Basenpaare ausgeschlossen sind, welche in der Reihenfolge, in der sie angeordnet sein würden, wenigstens ein »non-sens«-Codon (Abbruch-Codon) bilden würden.

2. Satz von Vectoren nach Anspruch 1, dadurch gekennzeichnet, daß die obgenannten drei Vectoren jeweils deutliche Mutationen (im Vergleich zueinander) aufweisen, welche ein rasches Erkennen der Vectoren bei genetischen Rekombinationsvorgängen, bei denen alle drei Vectoren zum Einsatz kommen, ermöglichen.

3. Satz von Vectoren nach Anspruch 1 oder nach Anspruch 2, dadurch gekennzeichnet, daß der obgenannte Promoter jener des Lactose-Operons ist und daß das obgenannte Gen das Z-Gen des gleichen Operons ist.

4. Satz von Vectoren nach Anspruch 3, dadurch gekennzeichnet, daß die obgenannte Erkennungsstelle für ein bestimmtes Restriktionsenzym eine EcoRI-Erkennungsstelle ist und daß in jedem dieser drei Vectoren nur eine einzige derartige EcoRI-Erkennungsstelle vorhanden ist.

5. Satz von Vectoren nach Anspruch 4, dadurch gekennzeichnet, daß in einem dieser Vectoren die Erkennungsstelle für das Restriktionsenzym vom Ausgangspunkt der Translation durch eine ganze Zahl von Tripletts, umfassend die ersten acht Tripletts, die den ersten acht Aminosäuren der $\beta$-Galactosidase entsprechen, und zu denen im zweiten Vector noch eine aus zwei Basenpaaren gebildete Gruppe und im dritten Vector noch ein oder vier Basenpaare hinzugefügt sind, getrennt ist.

6. Satz aus DNA-Fragmenten zur Herstellung eines Satzes von Vectoren nach einem beliebigen Anspruch der Ansprüche 1 bis 5, wobei diese Fragmente wenigstens den Promoter und einen Teil des Gens oder des einen der Gene, mit welchem er im entsprechenden bakteriellen Operon assoziiert ist, umfassen, und wobei diese Fragmente dadurch gekennzeichnet sind, daß sich zwei dieser Fragmente vom dritten durch eine Verschiebung ihrer Cohäsivtermini (Bindeenden) gegenüber dem Ausgangspunkt des Lesens des entsprechenden Gens oder Genteils unterscheiden, wobei diese Verschiebungen in den beiden Fragmenten durch Gruppen aus zwei Basenpaaren bzw. aus einem Basenpaar oder aus vier Basenpaaren erzeugt werden, mit welchen Basenpaaren gegebenenfalls außerdem noch ganze Zahlen von zusätzlichen Tripletts verbunden sein können, wobei die Basenpaare in diesen Gruppen von beliebiger Art sein können, jedoch unter Ausschluß von jenen Basenpaaren, welche in der Reihenfolge, in der sie angeordnet sein würden, wenigstens ein »non-sens«-Codon (Abbruch-Codon) bilden würden.

7. Satz aus DNA-Fragmenten nach Anspruch 6, dadurch gekennzeichnet, daß in jedem dieser Fragmente der Promoter und das entsprechende, mit ihm verbundene Gen vorzugsweise der Promoter bzw. das Z-Gen des Lactose-Operons sind, und daß die in Betracht gezogenen Cohäsivtermini EcoRI-Cohäsivtermini sind.

8. Satz aus DNA-Fragmenten nach Anspruch 7, dadurch gekennzeichnet, daß die erste Base des Cohäsivterminus von dem obgenannten Ausgangspunkt durch eine ganze Zahl von Tripletts, umfassend die ersten acht Tripletts, die ihrerseits den ersten acht Aminosäuren der $\beta$-Galactosidase entsprechen, und zu denen im zweiten Fragment noch eine aus zwei Basenpaaren gebildete Gruppe und im dritten Fragment noch ein Basenpaar oder vier Basenpaare hinzutreten, getrennt ist.

9. Verfahren zur aus einem wenigstens einen Cohäsivterminus, der einem bestimmten Restriktionsenzym entspricht, umfassenden Einbau-Fragment erfolgenden Herstellung von zwei anderen Fragmenten, deren Konstitutionen, nach Einbau in einen gleichen Vector, deutliche Phasenverschiebungen auf dem Niveau seiner Expression in einem Bakterium nach sich ziehen, wobei dieses Verfahren durch die folgenden Arbeitsgänge gekennzeichnet ist, welche darin bestehen,

— mittels einer geeigneten Endonuclease das hervorstehende Fragment, das einer der DNA-Stränge bildet, auf dem Niveau des obgenannten Cohäsivterminus zu schneiden,

— das auf diese Weise modifizierte Fragment mittels einer Ligase auf dem Niveau des gebildeten freien Cohäsivterminus (Bindeendes) mit einem bestimmten, unterschiedlichen, anderen DNA-Fragment zu rekombinieren, welches eine Erkennungsstelle für das gleiche Restriktionsenzym aufweist und ebenfalls ein freies Bindeende hat, wobei dieses freie Bindeende von dem ersten, für die Erkennungsstelle bestimmenden Basenpaar durch eine Gruppe von Basenpaaren getrennt ist, welche Gruppe umfaßt:

— entweder zwei Basenpaare und, gegebenenfalls, eine ganze Zahl von zusätzlichen Tripletts, die zu den vorhergehenden beiden Basenpaaren hinzugefügt sind,

— oder vier Basenpaare und, gegebenenfalls, eine ganze Zahl von zusätzlichen Tripletts, die zu den vorhergehenden vier Basenpaaren hinzugefügt sind; wobei die in dieser Gruppe enthaltenen Basenpaare von beliebiger Art sein können, jedoch unter Ausschluß von solchen Basenpaaren, welche in der Reihenfolge, in welcher sie angeordnet sein würden, wenigstens ein »nonsens«-Codon (Abbruch-Codon) bilden würden,

— das auf diese Weise erhaltene, modifizierte Einbau-Fragment mit diesem Restriktionsenzym zu behandeln, um erneut das in Betracht gezogene Bindeende zu bilden, und so das eine der beiden anderen Fragmente zu bilden, welches eine der obgenannten, deutlichen Phasenverschiebungen aufweist, und

— schließlich die vorstehend angeführten Arbeitsgänge mit dem erhaltenen Fragment zu wiederholen, um letztlich das andere der genannten Fragmente zu erhalten, welches die andere der genannten, deutlichen Phasenverschiebungen aufweist.

## Claims

1. Set of vectors comprising three vectors, in which each of these vectors contains a fragment composed at least of the promoter and at least of a part of the gene or of one of the genes associated with it in the corresponding bacterial operon, the point of initiation of the translation and a site of recognition by a specific restriction enzyme, this set being characterised essentially by the fact that the positions of this site of recognition are respectively displaced, in a second of these vectors, through the medium of a group comprising two pairs of bases and, if need be, a whole number of supplementary triplets, and, in a third of these vectors, through the medium of a group comprising one pair or four pairs of bases and, if need be, a whole number of supplementary triplets, with respect to the position of this site in the first of these vectors in relation to the aforesaid site of initiation, it being possible for the pairs of bases contained in these groups to be any whatsoever, with the exclusion, however, of those which, in the order in which they would be located, would form at least one »nonsense« codon.

2. Set of vectors according to claim 1, characterised in that the three aforesaid vectors comprise respectively different mutations permitting their rapid recognition in genetic recombination operations making use of all three.

3. Set of vectors according to claim 1 or claim 2, characterised in that the aforesaid promoter is that of the lactose operon and that the aforesaid gene is the Z gene of this same operon.

4. Set of vectors according to claim 3, characterised in that the aforesaid site of recognition by a specific restriction enzyme is an EcoRI site, this EcoRI site being unique in each of the said three vectors.

5. Set of vectors according to claim 4, characterised in that in one of these vectors the site of the restriction enzyme is separated from the point of initiation of the translation by a whole number of triplets comprising the first eight triplets corresponding to the first eight aminoacids of $\beta$-galactosidase, to which there is added in the second vector a group formed of two pairs of bases and in the third vector one pair or four pairs of bases.

6. Set of DNA fragments for making a set of vectors according to any one of claims 1 to 5, these fragments comprising at least the promoter and a part of the gene or of one of the genes associated with it in the corresponding bacterial operon, these fragments being characterised in that two of these fragments are distinguished from the third by a displacement of their cohesive ends with respect to the point of initiation of the reading of the corresponding gene or part of gene, these displacements being produced in the two fragments by groups of two pairs of bases and of one pair or four pairs of bases, respectively, with which, if need be, there may moreover be associated whole numbers of supplementary triplets, it being possible for the pairs of bases of these groups to be any whatsoever, with the exclusion, however, of those which, in the order in which they would be located, would form at least one »nonsense« codon.

7. Set of DNA fragments according to claim 6, characterised in that in each of them the promoter and the associated corresponding gene are preferably the promoter and the Z gene, respectively, of the lactose operon, the cohesive ends in question being cohesive EcoRI ends.

8. Set of DNA fragments according to claim 7, characterised in that the first base of the cohesive end is separated from the point of initiation aforesaid by a whole number of triplets comprising the first eight triplets, themselves corresponding to the first eight aminoacids of $\beta$-galactosidase, to which there are added, in the second fragment, a group formed of two pairs of bases and, in the third fragment, of one pair or four pairs of bases.

9. Process for producing from an insertion fragment comprising at least one cohesive end corresponding to a specific restriction enzyme, another two fragments whose compositions cause, after insertion in the same vector, different displacements of phase at the level of its manifestation in a bacterium, this process being characterised by the operations which consist in:

— trimming the salient fragment which forms one of the strands of the DNA at the level of the said cohesive end by means of a suitable endonuclease,

— recombining, by means of a ligase, the fragment modified in this way, at the level of the free end formed, with another given different DNA fragment having a site of recognition for this same restriction enzyme and also comprising a free end, this being separated from the first pair of bases intervening in the site of recognition by a group of pairs of bases containing

— either two pairs of bases and, if need be, a whole number of supplementary triplets adding themselves to the foregoing two pairs of bases,

— or four pairs of bases and, if need be, a whole number of supplementary triplets adding themselves to the foregoing four pairs of bases; it being possible for the pairs of bases contained in this group to be any whatsoever, with the exclusion, however, of those which, in the order in which they would be located, would form at least one »nonsense« codon,

— treating the modified insertion fragment obtained in this way with a restriction enzyme so as to reform the cohesive end in question and form in this way one of the other two fragments comprising one of the aforesaid different displacements of phase, and

— finally, repeating the foregoing operations on the fragment obtained in order finally to obtain the other of the said fragments comprising the other of the said different displacements of phase.

Fig.1.

Fig.1a.

Fig.1b.

Fig.1c.

Fig.1d.

Fig.2.

Fig.2a.

Fig.2b.

Fig.2c.

Fig.3.

# Fig.4.

Fig.4a.

Fig.4b.

Fig.4c.

Fig.4d.